# EUROPÄISCHE PATENTSCHRIFT

(11) **EP 0 863 723 B1**
(45) Veröffentlichungstag und Bekanntmachung des Hinweises auf die Patenterteilung: **18.04.2001**
(21) Anmeldenummer: 95937987.6
(22) Anmeldetag: 01.12.1995
(51) Int. Cl.: A61B 10/00, A61J 17/00

(54) **VORRICHTUNG ZUR ENTNAHME VON SPEICHELPROBEN**
SALIVA SAMPLING DEVICE
DISPOSITIF DE PRELEVEMENT D'ECHANTILLONS DE SALIVE

(43) Veröffentlichungstag der Anmeldung: 16.09.1998
(73) Patentinhaber: Lamprecht AG, 8050 Zürich (CH)
(72) Erfinder: SUHONEN, Jouko, CH-8302 Kloten (CH)
(74) Vertreter: Blum, Rudolf Emil Ernst
(86) Internationale Anmeldenummer: IB9501084
(87) Internationale Veröffentlichungsnummer: WO9720502

(56) Entgegenhaltungen:
- WO-A-95/30484
- US-A- 3 610 248

## Beschreibung

Die vorliegende Erfindung betrifft eine Vorrichtung zur Entnahme von Speichelproben für medizinische Untersuchungen.

Speichel kann bekanntlich dazu verwendet werden, Absonderungen von Arzneimitteln, Hormonen und Antikörper (z.B. HIV-Antikörper) mit Speichel festzustellen und zu überwachen. Gegenwärtig ist es beispielsweise möglich, alle Steroide mit einer diagnostischen Bedeutung in einer routinemässigen klinischen Endokrinologie im Speichel sehr einfach zu messen. Speichel ist auch insbesondere wertvoll für auf einer breiten Basis angelegte Untersuchungen und für epidemiologische Studien. Zur Ueberwachung von vielen subklinischen und klinischen Zuständen kann Speichel anstelle von Blut verwendet werden. Der Eintritt von Arzneimitteln in den Speichel erfolgt gemäss den allgemeinen Grundlagen der Bewegung von Arzneimitteln durch biologische Membranen. Die nicht gebundene Fraktion des Arzneimittels im Plasma steht für die Diffusion in den Speichel zur Verfügung. Die Vorteile von Speichelproben im pädriatischen und neonatalogischen Bereich sind offensichtlich, weil eine Probeentnahme über den Speichel schmerzlos ist und das Blut schont. Studien an Kindern empfehlen Speichel zum therapeutischen Ueberwachen des Phenytoins, Carbamazepins und Phenobarbitals. Speichelproben für ein diagnostisches und therapeutisches Ueberwachen ist bei Neugeborenen und Frühgeburten vielversprechend. Einige Erkrankungen des Systems wie z.B. das Sjörgen Syndrom, zystische Fibrose und Erkrankungen der adrenalen Cortex können die Funktionen der Speicheldrüsen beeinflussen, bei welchen Erkrankungen Speichel als diagnostische Hilfe verwendet werden kann. Die Liste von medizinischen Zus-tänden, welche in der oralen Flüssigkeit als diagnostisches Medium überprüft und festgestellt werden können, wächst dauernd.

Weiter werden Pathogene im Speichel (Erkrankungen verursachende Bakterien) als diagnostische Werkzeuge verwendet, um bei Personen festzustellen, ob ein Risiko verschiedener oraler Erkrankungen vorhanden ist, beispielsweise Karies der Zähne und Periondontitis. Pathogene wie mutans streptokokken, Actinobacillus actinomycetemcomitans, B. gingivalis, F. nucleatum, etc. spielen eine wichtige Rolle in bezug auf die Entstehung von Munderkrankungen. Weil die Kolonisation von Pathogenen im Mund und auf den Zähnen des Kleinkindes offensichtlich das Risiko einer zukünftigen Erkrankung in sich birgt, ist es wichtig, anfällige Personen so früh als möglich zu ermitteln, vorzugsweise noch im Kindesalter. Die Zählung von Bakterien im Speichel offenbart direkt das Ausmass der Kolonisation im Zielgewebe des Mundes. In der nahen Zukunft wird das Probeentnehmen von Bakterien im Speichel ein routinehaftes Vorgehen bei Untersuchungen von Mutter-Kind-Paaren und der Überwachung der Gesundheit allgemein sein. Je früher das Eindringen von Pathogenen ermittelt wird, desto früher können Gegenmassnahmen getroffen werden und es können orale Behandlungen verstärkt werden, z.B. indem spezifische Antikörper gegen diese Pathogene verwendet werden.

Währenddem nun bei erwachsenen Personen die Entnahme, bzw. das Sammeln von Speichel keine Schwierigkeiten bietet, ist dies bei Neugeborenen, bei Säuglingen und Kleinkindern praktisch unmöglich.

Die Abgabe von Speichel ist bei erwachsenen Personen ein auf eine Aufforderung hin erfolgender Willensakt, und nur schon das Oeffnen des Mundes erfolgt auf eine Aufforderung hin.

Ein solcher Vorgang ist bei Neugeborenen und Kleinkindern unmöglich. Weiter akzeptiert das Kind keine für es gefühlsmässigen Fremdkörper, und eine direkter Kontakt zwischen einem saugförmigen Speichelabsorptionskörper und dem Mund, der Zunge, etc. des Kindes ist unbedingt zu vermeiden, insbesondere auch zum Verhindern, dass sich vom Speichelabsorptionskörper Bruchstücke abtrennen können und eine erhebliche Gefährdung des Kindes verursachen könnten.

Die WO-A-95/30484 zeigt eine Vorrichtung zur Entnahme von Speichelproben, welche einen als Speichlaufnahmekörper dienenden, zylinderförmigen Schwammteil aufweist, der in den Mund einer jeweiligen Person zu plazieren ist, um damit Speichel aufzusaugen. Ein solcher Schwammteil wird jedoch von einem Kleinkind nicht akzeptiert, weil es denselben seiner Aussenform nach als Fremdkörper empfindet. Weiter weist der Schwammteil eine strukturierte, offenporige Oberfläche auf, so dass der Schwammteil vom Kind noch vermehrt als Fremdkörper empfunden wird. Weiter besteht ein direkter Kontakt zwischen dem Schwammteil und der Mundhöhle, so dass vom Schwammteil Bruchstücke abgetrennt werden können, die eine erhebliche Gefährdung des Kindes verursachen könnten.

Die US-A-3 610 248, nach der die zweiteilige Form der unabhängigen Ansprüche 1 und 4 abgegrenzt ist, offenbart eine Kau- und Beissvorrichtung für Kleinkinder, insbesondere im Stadium des Zahnens. Diese Kau- und Beissvorrichtung weist einen hohlen, keulenförmigen Kaukörper mit einer perforierten Wand auf, in welchen ein hydrophiler, den Innenraum des hohlen Kaukörpers vollständig ausfüllender Füllkörper eingesetzt ist. Dieser Füllkörper lässt sich jedoch nicht aus dem Kaukörper entfernen um eine Speicheluntersuchung zu ermöglichen. Weiter dient der Füllkörper ausschliesslich dazu, Wirkstoffe unter dem Einfluss von Speichel freizugeben, so dass sie in die Mundhöhle eindringen können, jedoch nicht dazu, Speichel aus der Mundhöhle aufzunehmen.

Ziel der Erfindung ist allgemein eine Vorrichtung zur sicheren Entnahme von Speichelproben eines Frühgeborenen, Neugeborenen und Kleinkindes zu schaffen.

Ein weiteres Ziel ist eine Vorrichtung zur Entnahme von Speichelproben zu schaffen, die einen Hohlkörper zur Aufnahme eines daraus entfernbaren, Flüssigkeiten absorbierenden Gegenstandes und ein Verschlussglied aufweist, welcher Hohlkörper aus einem nachgiebigen Material besteht, mindestens eine Perforation für den Durchtritt von Speichel und eine durch das Verschlussglied flüssigkeitsdicht verschliessbare Oeffnung zum Einsetzen, bzw. Entnehmen des Flüssigkeiten absorbierenden Gegenstandes aufweist.

Erfindungsgemässe Vorrichtungen sind durch die Merkmale der unabhängigen Ansprüche 1 oder 4 gekennzeichnet. Vorteilhafte Ausführungen ergeben sich aus den abhängigen Ansprüchen.

Die durch die Erfindung erreichten Vorteile sind insbesondere darin zu sehen, dass mittels der Vorrichtung Speichel gefahrlos von einem Frühgeborenen, Neugeborenen und Kleinkind entnommen werden kann und dass dem Hohlkörper eine vom Kind im Mund angenommene Form gegeben werden kann.

Weitere Ziele und Vorteile ergeben sich aus der nachfolgenden Beschreibung, anhand welcher die Erfindung anhand von Ausführungswegen darstellender Zeichnungen beispielsweise näher erläutert wird.

Es zeigt:
Figur 1 einen vertikalen Schnitt durch einen Schnuller zur Entnahme von Speichelproben;
Figur 2 einen horizontalen Schnitt durch den rechten Endbereich des Schnullers nach Figur 1;
Figur 3 eine Seitenansicht lediglich des Saugkörpers des Schnullers;
Figur 4 eine Ansicht von hinten auf den Saugkörper des Schnullers;
Figur 5 eine Variante der Form der Durchtrittslöcher im Saugkörper;
Figur 6 eine weitere Variante der Form der Durchtrittslöcher im Saugkörper;
Figur 7 in einem vergrösserten Massstab einen Ausschnitt der Figur 1, wobei zur Verdeutlichung die einzelnen Bauteile in einem Abstand voneinander gezeichnet sind; und
Figur 8 in einem vergrösserten Massstab die Figur 2, wobei zur Verdeutlichung die einzelnen Bauteile in einem Abstand voneinander gezeichnet sind,
Figur 9 eine weitere Ausführung der erfindungsgemässen Vorrichtung, und
Figur 10 eine weitere Variante des Schnullers.

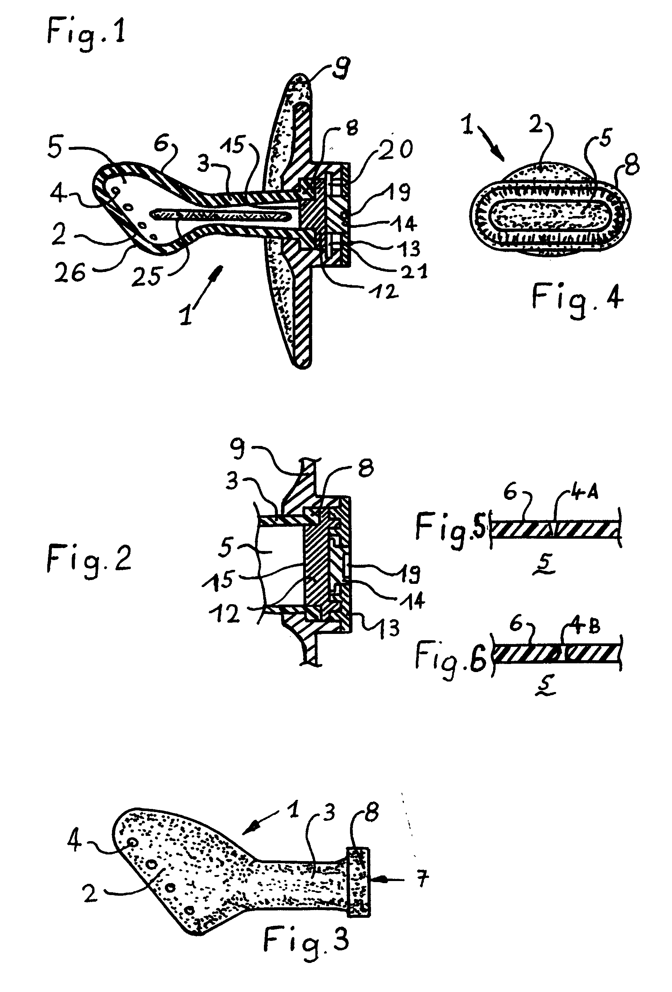

Der als Hohlkörper ausgebildete Saugkörper 1, der schematisch in der Figur 1 im Schnitt, in der Figur 3 in der Seitenansicht und in Figur 4 in einer Ansicht von hinten dargestellt ist, weist einen vorderen, ersten freien Endabschnitt 2 und einen daran anschliessenden hinteren, zweiten Endabschnitt 3 auf.

Der aus einem elastischen Material hergestellte Saugkörper 1 weist die Form und Elastizität des Saugkörpers herkömmlicher Schnuller auf. Es ist eine kieferorthopädisch günstige, die kaufunktionelle Leistungsfähigkeit und das Kieferwachstum fördernde Form. Ein Schnuller mit einem solchen Saugkörper ist beispielsweise in der Schweizer Patentschrift CH-PS(bzw. CH-A)-495 748 offenbart.

Im vorderen, ersten Endabschnitt 2 sind Perforationen in Form von Durchtrittslöchern 4 angeordnet, die den Innenraum 5 des insgesamten Saugkörpers 1 mit seiner Aussenfläche 6, also mit der Umgebung verbinden. Auf diese Perforationen, i.e. Durchtrittslöcher 4 wird weiter unten im einzelnen eingegangen.

Beim vom ersten Endabschnitt 2 entfernten Bereich endet der zweite Endabschnitt 3 des Saugkörpers 1 bei einer Oeffnung, nachfolgend als Entnahmeöffnung 7 bezeichnet (siehe Fig. 3). Diese Entnahmeöffnung 7 ist von einem Umfangswulst 8 umgeben.

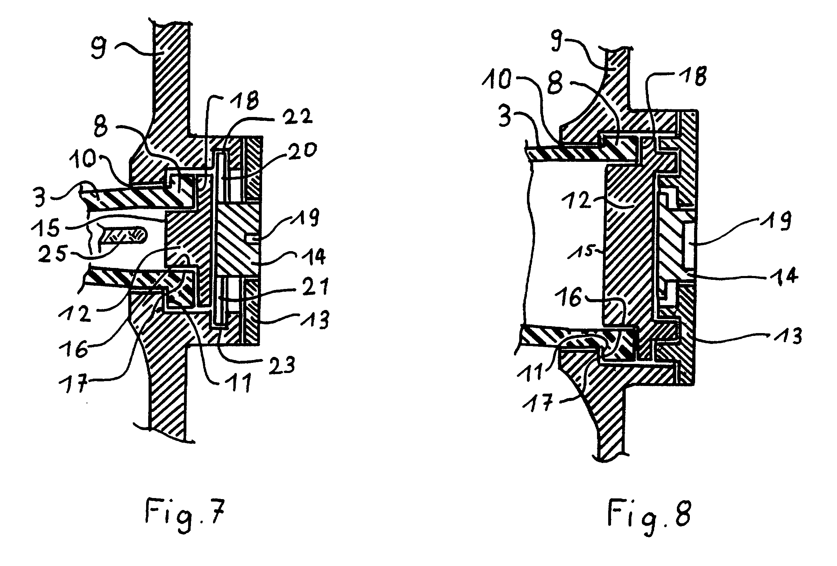

Der Saugkörper 1 ist in einem Schild 9 eingesetzt. Dieser Schild 9 ist bei der gezeichneten Ausführung gewölbt ausgebildet, wie bei Schnullern allgemein bekannt ist. Offensichtlich sind auch andere Formen denkbar, beispielsweise nach aussen gewölbte oder ebenflächige Ausbildungen. Der Schild 9 weist eine mittige Oeffnung 10 auf (Fig. 7 und 8), in welcher der zweite Endabschnitt 3 des Saugkörpers 1 eingesetzt ist. Es ist ersichtlich, dass die dem zweiten Endabschnitt 3 zugekehrte Ringfläche 11 des Umfangswulstes 8 dichtend an der entsprechenden Gegenfläche des Schildes 9 anliegt.

Im Schild 9 ist ein Verschlussglied eingesetzt, welches einen Innenteil 12, einen Aussenteil 13 und ein im Aussenteil 13 drehbar gelagertes Riegelglied 14 enthält. Der Innenteil 12 ist mittels einer Klemmverbindung mit dem Aussenteil 13 verbunden, wie insbesondere aus der Fig. 2 ersichtlich ist. Bei seinem Innenteil 12 weist das Verschlussglied einen Vorsprungsabschnitt 15 auf. Dieser Vorsprungsabschnitt 15 ragt in die Entnahmeöffnung 7 (Fig. 3) des Saugkörpers 1 hinein. Die Querschnittsform des Vorsprungsabschnittes 15 entspricht der Querschnittsform der Entnahmeöffnung 7. Damit liegt der Vorsprungsabschnitt 15 mit seiner Aussenumfangsfläche 16 an einem entsprechenden Innenflächenabschnitt 17 des zweiten Endabschnittes 3 des Saugkörpers 1 an. Schliesslich weist das Verschlussglied bei einem Ende des Vorsprungsabschnittes 15 des Innenteils 12 einen Schulterabschnitt 18 auf, der dichtend am Umfangswulst 8 anliegt.

Im Aussenteil 13 des Verschlussgliedes ist das Riegelglied 14 drehbar gelagert. Das Riegelglied 14 weist aussen eine schlitzförmige Vertiefung 19 zur Aufnahme eines beispielsweise schraubenzieherförmigen Werkzeugteils zum Rotieren des Riegelgliedes 14 auf. Das Riegelglied 14 enthält weiter zwei plattenförmige Vorsprünge 20,21, welche in entsprechende Ausnehmungen 22,23 im Schild 9 eingreift.

Es ist somit ersichtlich, dass das Verschlussglied 12,13,14 über sein Riegelglied 14 lösbar mit dem Schild 9 verbunden ist und im arretierten Zustand der Umfangswulst 8 des Saugkörpers 1 dichtend und festgeklemmt zwischen dem Schild 9 und dem Verschlussglied 12,13,14 gehalten ist.

Im Saugkörper 1 sind Durchtrittslöcher 4 als Perforationen angeordnet. In der in den Figuren 1 und 3 gezeigten Ausführung sind diese Durchtrittslöcher 4 nur beim vorderen, ersten Endabschnitt 2 vorhanden, und entlang des gezeichneten, ebenflächigen Bereiches 26 dieses ersten Endabschnittes 2 verteilt angeordnet. Gemäss einer anderen (nicht gezeichneten) Ausführung ist lediglich ein Durchtrittsloch im Zentrum des genannten ebenflächigen Bereiches 26 und ein weiteres Durchtrittsloch bei der gegenüberliegenden, also schwach gewölbten Wand des ersten Endabschnittes angeordnet. Die Mittelachsen der Durchtrittslöcher müssen nicht unbedingt rechtwinklig zu den betreffenden Wandabschnitten des ersten Endabschnittes 2 verlaufen. Sie können auch schiefwinklig zu den Wandabschnitten gegen den Innenraum des zweiten Endabschnittes 3 hin gerichtet verlaufen. Weiter müssen diese Durchtrittslöcher 4 nicht eine kreisrunde Querschnittsform aufweisen. Die Querschnittsform kann gemäss weiteren Ausführungen langgestreckt, z.B. schlitzförmig sein. Auch können weitere Durchtrittslöcher im zweiten Endabschnitt 3 ausgebildet sein. Zur Bildung der vorgeschriebenen Perforationen müssen nicht unbedingt Durchtrittslöcher oder Schlitze vorhanden sein. Als weitere vorgesehene Ausführung kann der Saugkörper 1, bzw. mindestens der vordere Endabschnitt die Form eines Netzes oder Gewebes aufweisen.

Die Durchtrittslöcher 4, Schlitze, etc. können eine zylinderförmige Innenumfangswand aufweisen. Gemäss der Ausführung nach Figur 5 sind die Durchtrittslöcher 4 in Richtung zum Innenraum 5 zusammenlaufend ausgebildet. Damit wird einem Rückfluss vom Innenraum 5 nach aussen entgegengewirkt. Eine mindestens teilweise Berührung der beim Innenraum 5 gelegenen Ränder der Durchtrittslöcher ist auch möglich. Gemäss einer weiteren Ausführung, in Figur 6 gezeigt, sind die Innenwände der Durchtrittslöcher 4 gegen die Aussenfläche 6 und die Innenfläche 24 des Saugkörpers 1 abgerundet ausgebildet.

Gemäss einer weiteren vorgesehenen Ausführung ist bei allen oder bei mindestens einem Teil der Perforationen ein kleines Schlauchstück vorhanden, das von der Innenwand des Saugkörpers 1 gegen den Innenraum derselben ragt. Diese kleinen Schlauchstücke tragen dazu bei, einem Rückströmen des in den Innenraum eingeflossenen Speichels durch die Perforationen zurück in die Mundhöhle entgegenzuwirken.

Im Innenraum 5 des Saugkörpers 1 ist der Flüssigkeiten absorbierende Gegenstand 25 angeordnet. Dieser weist die Form eines langgestreckten Plättchens auf. Allgemein kann er ein osmotisches Gebilde sein, das ein genügend grosses Volumen eines reinen Ultrafiltrates ohne Moleküle grösser als 12'000 dalton sammelt. Auch andere Arten von Absorbtionsgebilden mit einem guten Absorptionsvermögen und einer guten Aufnahmefähigkeit, beispielsweise eine inerte medizinische Watte, Nonwoven-Materialien, (Fibrella)-Rollen/Kissen, Schaumstoff, alles Materialien, die sterilisiert werden können, sind vorgesehen.

Der Speichelfluss kann weiter durch im Absorptionsgebilde angeordnete Zitronensäure, Xylitol oder andere zweckdienlichen Anregungsmitteln angeregt werden. Eine solche Stimulation ist vorteilhaft für die analytische Verwendbarkeit von Mundflüssigkeit für gewisse kleine Moleküle, jedoch nicht für grosse Moleküle. Wenn muko- und gingivale Transudate, die grosse Moleküle enthalten, überprüft und überwacht werden sollen, und die z.B. für die Diagnose von Infektionskrankheiten wichtig sind, sollte das Sammeln von Speichel ohne Verwendung von Stimulantien durchgeführt werden.

Vorteilhaft wird ein Farbindikator verwendet der sich dann blau oder zu einer anderen Farbe verfärbt, wenn das inerte osmotische Gebilde, bzw. das Absorptionsgebilde für ein weiteres Verarbeiten genügend befeuchtet ist. Solche Farbindikatoren sind allgemein bekannt. Gemäss einer weiteren Ausbildung sind das Riegelglied 14 und mindestens ein Teil des Vorsprungabschnittes 15 aus einem mölglichst durchsichtigen Werkstoff gebildet, so dass zur Feststellung der Verfärbung der Schnuller nicht aus dem Mund entfernt werden muss.

Zur Entnahme einer Speichelprobe wird dem Kleinkind der beschriebene Schnuller anstelle des üblichen Schnullers, an welchem das Kleinkind saugt, in den Mund gesteckt. Da die Form des Saugkörpers 1 der Form des Saugkörpers bekannter, gewöhnlicher Schnuller entspricht (der Saugkörper muss nicht unbedingt die aus den Zeichnungsfiguren ersichtliche Form aufweisen), und an welchen das Kind gewöhnt ist, wird der Austausch vom Kind nicht wahrgenommen, d.h. dass die schnullerförmige Vorrichtung zur Entnahme der Speichelprobe vom Kleinkind akzeptiert wird. Befindet sich der Saugkörper im Mund, fliesst nun der Speichel durch die Durchtrittslöcher 4 hindurch in den Innenraum 5 des Saugkörpers 1 und befeuchtet das darin angeordnete aufnahmefähig Gebilde 25. Bei intermittierenden Saugbewegungen des Kleinkindes kann mindestens der vorderste, erste Endabschnitt 2 des Saugkörpers 1 geringen Formveränderungen ausgesetzt werden, oder es kann im Innenraum 5 ein intermittierender, kleiner Unterdruck entstehen, so dass der sich auf der Aussenfläche des Saugkörpers 1 befindliche Speichel grundsätzlich in den Innenraum 5 hineingepumpt wird.

Nach einigen Minuten, bzw. nachdem sich der genannte Farbindikator verfärbt hat, wird der Schnuller aus dem Mund entfernt und dem Kind wieder der vorgängige Schnuller gegeben.

Danach wird durch das Drehen des Riegelgliedes 14 das gesamte Verschlussglied 12-14 vom Schild 9 gelöst. Mit beispielsweise einer Pinzette wird das nun mit Speichel getränkte plattenförmige Gebilde 25 aus dem Saugkörper 1 entfernt. Darauf wird der Speiches aus dem getränkten Gebilde 25 mittels einer zweckdienlichen Vorrichtung in ein Reagenzglas, eine Schale, einen Becher z.B. zum sofortigen Verarbeiten und Untersuchen, oder auch in einen an sich bekannten Versandbehälter, wenn die Untersuchung nicht am selben Ort durchgeführt werden kann, ausgepresst, um unmittelbar bzw. später analysiert zu werden. Das Auspressen des Speichels kann mit unterschiedlichen Geräten durchgeführt werden. Es kann z.B. ein zangenförmiges Werkzeug verwendet werden. Eine weitere vorgesehene Vorrichtung ist eine Wegwerfspritze (ohne Injektionsnadel). Das mit dem Speichel getränkte Gebilde 25 wird bei herausgenommenem Kolben in den Innenraum des zylinderförmigen Spritzenkörpers eingebracht, danach der Verschlussteil der Spritze mit dem Kolben aufgesetzt und der Speichel mittels dem Kolben aus dem Gebilde 25 in den jeweiligen Aufnahmebehälter ausgepresst.

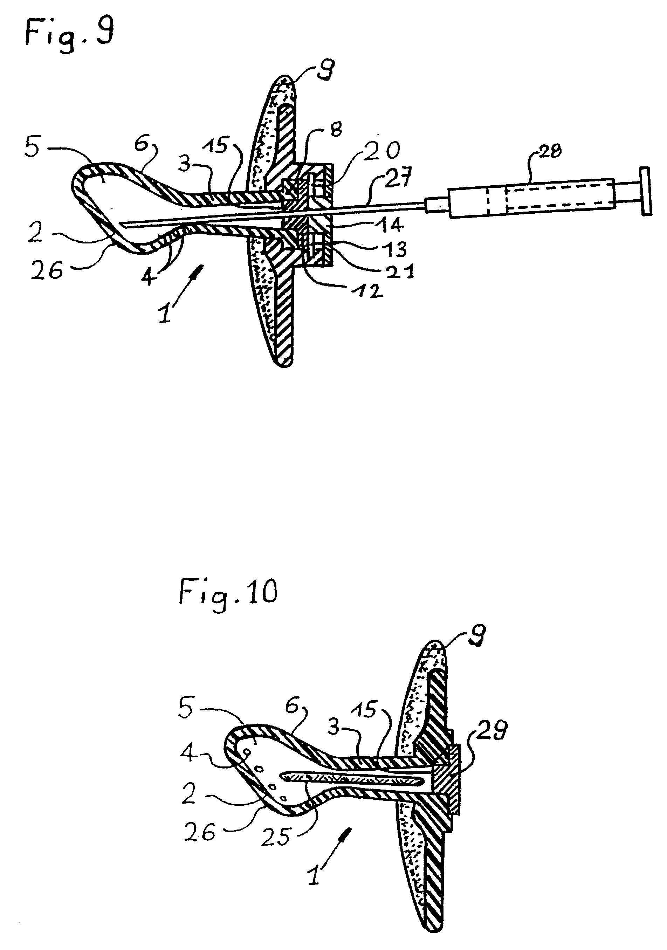

Die Figur 9 zeigt eine weitere Ausführung der erfindungsgemässen Vorrichtung. Dabei sind für dieselben Bauteile und Abschnitte dieselben Bezugsziffern wie im vorgängig beschriebenen Beispiel verwendet. Im Vergleich insbesondere mit der Ausführung nach Figur 1 ist im Saugkörper 1 kein Flüssigkeiten absorbierender Gegenstand 25 angeordnet. Weiter sind die Durchtrittslöcher 4 nicht beim vordersten Endbereich des Saugkörpers 1 angeordnet, sondern, wie aus der Figur 9 hervorgeht, in einem Abstand vom vorderen Endbereich.

Ein Schlauchstück 27, bevorzugt eine Mikropipette erstreckt sich von hinten in den Saugkörper 1 und endet kurz vor dessen vorderer Abschlusswand. Diese Mikropipette 27 ist mit einem Aspirator 28 einer an sich bekannten Ausbildung verbunden.

Die Wirkungsweise dieser Ausführung ist wie folgt: Der durch die Durchtrittslöcher 4 einströmende Speichel sammelt sich im vorderen Raumabschnitt des Saugkörpers 1, bei welchem keine Durchtrittslöcher 4 vorhanden sind und wo die Mikropipette endet. Nach dem sich eine genügende Menge Speichel in diesem Raum angesammelt hat, kann dieser Speichel durch eine Betätigung des Aspirators 28 aus dem Saugkörper 1 entnommen und wie eingangs erwähnt klinisch analysiert werden.

Figur 10 zeigt eine Ausführungsvariante des Schnullers, bei welcher insbesondere der Saugkörpers 1 und das Schild 9 aus einem gegossenen Teil bestehen. Hinten wird der Saugkörper 1 durch eine einfache Klappe 29 abgeschlossen, die am Schild 9 ausgelenkt ist. Diese Ausführungsvariante lässt sich sowohl mit einem Flüssigkeiten absorbierenden Gegenstand 25 nach Figur 1 als auch mit einer Mikropipette 27 und daran angeschlossenen Aspirator nach Figur 9 verwenden. Beispielsweise gezeigt ist die Variante nach Figur 1.

## Patentansprüche

1. Vorrichtung zur Entnahme von Speichelproben, einschliesslich einem Hohlkörper (1) mit einem Innenraum (5) zum Sammeln von Speichel, welcher Hohlkörper (1) aus einem nachgiebigen Material besteht und mindestens eine Perforation (4, 4A, 4B) für den Durchtritt von Speichel von aussen in den Innenraum (5) aufweist, in welchem Hohlkörper (1) ein Aufnahmeteil (27) zur Aufnahme und zum Entfernen des in den Innenraum (5) eingetretenen Speichels angeordnet ist, welcher Hohlkörper (1) die Form des Saugkörpers eines Schnullers aufweist und mit einem Schild (9) in Verbindung steht, in welchem Schild (9) ein Verschlussglied (12,13,14,29) lösbar arretiert angeordnet ist, das im arretierten Zustand den Innenraum (5) des Saugkörpers gegen die Umgebung absperrt, dadurch gekennzeichnet, dass der Saugkörper einen ersten, freien Endabschnitt (2) und einen zweiten, dem ersten entgegengesetzt und beim Schild (9) angeordneten Endabschnitt (3) aufweist, dass der Aufnahmeteil zur Aufnahme und zum Entfernen des in den Innenraum (5) eingetretenen Speichels ein Abschnitt eines Schlauchstückes (28) ist, das bei einem Ende im ersten Endabschnitt (2) endet und beim entgegengesetzten Ende durch das Verschlussglied (12,13,14,29) hindurch verläuft und zur Verbindung mit einer Absaugvorrichtung (28) für den gesammelten Speichel ausgebildet ist, und dass Perforationen (4) nur im zweiten Endabschnitt (3) des Saugkörpers angeordnet ist.

2. Vorrichtung nach Anspruch 1, dadurch gekennzeichnet, dass eine Mehrzahl Perforationen (4,4A) in Form von Durchtrittslöchern vorhanden ist, wobei jedes Durchtrittsloch in Richtung zum Innenraum (5) zusammenlaufend ausgebildet ist.

3. Vorrichtung nach Anspruch 1, dadurch gekennzeichnet, dass eine Mehrzahl Perforationen (4,4B) in Form von Durchtrittslöchern vorhanden sind, wobei jedes Durchtrittsloch eine Innenumfangswand aufweist, die gegen die Aussen- und Innenfläche des Saugkörpers (1) hin abgerundet ausgebildet ist.

4. Vorrichtung zur Entnahme von Speichelproben, einschliesslich einem Hohlkörper (1) mit einem Innenraum (5) zum Sammeln von Speichel, welcher Hohlkörper (1) aus einem nachgiebigen Material besteht und mindestens eine Perforation (4, 4A, 4B) für den Durchtritt von Speichel von aussen in den Innenraum (5) aufweist, in welchem Hohlkörper (1) ein Aufnahmeteil (25) zur Aufnahme und zum Entfernen des in den Innenraum (5) eingetretenen Speichels angeordnet ist, welcher Hohlkörper (1) die Form des Saugkörpers eines Schnullers aufweist und mit einem Schild (9) in Verbindung steht, in welchem Schild (9) ein Verschlussglied (12,13,14,29) lösbar arretiert angeordnet ist, das im arretierten Zustand den Innenraum (5) des Saugkörpers gegen die Umgebung absperrt, dadurch gekennzeichnet, dass der Aufnahmeteil zur Aufnahme und zum Entfernen des in den Innenraum eingetretenen Speichels ein aus dem Saugkörper entfernbarer, Flüssigkeiten absorbierender Gegenstand (25) ist.

5. Vorrichtung nach Anspruch 4, dadurch gekennzeichnet, dass eine Mehrzahl Perforationen (4,4A) in Form von Durchtrittslöchern vorhanden sind, wobei jedes Durchtrittsloch in Richtung zum Innenraum (5) zusammenlaufend ausgebildet ist.

6. Vorrichtung nach Anspruch 4, dadurch gekennzeichnet, dass eine Mehrzahl Perforationen (4,4B) in Form von Durchtrittslöchern vorhanden sind, wobei jedes Durchtrittsloch eine Innenumfangswand aufweist, die gegen die Aussen- und Innenfläche des Saugkörpers (1) hin abgerundet ausgebildet ist.

## Claims

1. Device for getting saliva samples, comprising a hollow body (1) having an inner space (5) for collecting saliva, where the hollow body (1) consists of a flexible material and presents at least one perforation (4, 4A, 4B) for the passage of saliva from outside into the inner space (5) which contains a retaining body (27) for taking up the saliva that has entered the inner space (5) and for removing it from the same, the hollow body (1) being shaped like the nipple of a teat and connected to a shield (9) that contains removably attached locking means (12, 13, 14, 29) which when locked do separate the inner space (5) of the nipple from the surroundings, characterized in that the nipple comprises a first free end section (2) and a second end section (3) opposite to the first and placed near the shield (9), the retaining body being a section (28) of a hose one extremity of which ends at the first end section and the other end of which passes through the locking means (12, 13, 14, 29) and is shaped for being connected to a sucking device (28) for the collected saliva, and in that the perforations (4) are situated only in the second end section (3).

2. Device according to claim 1, characterized in that it comprises several perforations (4, 4A) in the shape of passage holes, each of which constricts towards the inner space (5).

3. Device according to claim 1, characterized in that it comprises several perforations (4, 4A) in the shape of passage holes, each of which has an inner peripheral wall that is rounded towards the outer and the inner surfaces of the nipple (1).

4. Device for getting saliva samples, comprising a hollow body (1) having an inner space (5) for collecting saliva, where the hollow body (1) consists of a flexible material and presents at least one perforation (4, 4A, 4B) for the passage of saliva from outside into the inner space (5) which contains a retaining body (25) for taking up the saliva that has entered the inner space (5) and for removing it from the same, the hollow body (1) being shaped like the nipple of a teat and connected to a shield (9) that contains removably attached locking means (12, 13, 14, 29) which when locked do separate the inner space (5) of the nipple from the surroundings, characterized in that the retaining body is a liquid-absorbing object (25) removable from the nipple.

5. Device according to claim 4, characterized in that it comprises several perforations (4, 4A) in the shape of passage holes, each of which constricts towards the inner space (5).

6. Device according to claim 4, characterized in that it comprises several perforations (4, 4A) in the shape of passage holes, each of which has an inner peripheral wall that is rounded towards the outer and the inner surfaces of the nipple (1).

## Revendications

1. Dispositif pour prélever des échantillons de salive, avec un corps creux (1) ayant un espace intérieur (5) pour accumuler la salive, le corps creux (1) consistant en un matériau flexible et comportant au moins une perforation (4, 4A, 4B) pour le passage de la salive de l'extérieur vers l'espace intérieur (5), un organe récepteur (27) étant agencé dans le corps creux (1) pour recevoir la salive ayant pénétré dans l'espace intérieur (5) et pour l'extraire de celui-ci, le corps creux (1) ayant la forme du téton d'une tétine et étant réuni à un bouclier (9) dans lequel est bloqué de façon amovible un verrou (12, 13, 14, 29) qui en sa position verrouillée sépare l'espace intérieur (5) d'avec l'environnement, caractérisée en ce que le téton comprend une première partie extrémale libre (2) et une seconde partie extrémale (3) disposée à l'opposé de la première et près du bouclier (9), que l'organe récepteur (5) est une section d'un morceau de tuyau (28) se terminent à un bout dans la première partie extrémale (2), et passant à son bout opposé à travers le verrou (12, 13, 14, 29), ce bout étant agencé pour être connecté avec un dispositif (28) d'aspiration de la salive collectée, et en ce que les perforations (4) sont disposées exclusivement dans la seconde partie extrémale (3) du téton.

2. Dispositif selon la revendication 1, caractérisée en ce qu'il comporte plusieurs perforations (4, 4A) formant des trous de passage, chaque trou de passage se rétrécissant en direction de l'espace intérieur (5).

3. Dispositif selon la revendication 1, caractérisée en ce qu'il comporte plusieurs perforations (4, 4A) formant des trous de passage, chaque trou de passage présentant une paroi périphérique intérieure arrondie vers les surfaces extérieure et intérieure du corps creux (1).

4. Dispositif pour prélever des échantillons de salive, avec un corps creux (1) ayant un espace intérieur (5) pour accumuler la salive, le corps creux (1) consistant en un matériau flexible et comportant au moins une perforation (4, 4A, 4B) pour le passage de la salive de l'extérieur vers l'espace intérieur (5), un organe récepteur (25) étant agencé dans le corps creux (1) pour recevoir la salive ayant pénétré dans l'espace intérieur (5) et pour l'extraire de celui-ci, le corps creux (1) ayant la forme du téton d'une tétine et étant réuni à un bouclier (9) dans lequel est bloqué de façon amovible un verrou (12, 13, 14, 29) qui en sa position verrouillée sépare l'espace intérieur (5) d'avec l'environnement, caractérisée en ce que l'organe récepteur est un objet (25) absorbant les liquides et agencé pour pouvoir être extrait du téton.

5. Dispositif selon la revendication 4, caractérisée en ce qu'il comporte plusieurs perforations (4, 4A) formant des trous de passage, chaque trou de passage se rétrécissant en direction de l'espace intérieur (5).

6. Dispositif selon la revendication 4, caractérisée en ce qu'il comporte plusieurs perforations (4, 4A) formant des trous de passage, chaque trou de passage présentant une paroi périphérique intérieure arrondie vers les surfaces extérieure et intérieure du corps creux (1).
